# EUROPÄISCHE PATENTANMELDUNG

(11) **EP 2 314 286 A1**
(43) Veröffentlichungstag der Anmeldung: **27.04.2011**
(21) Anmeldenummer: 09013308.3
(22) Anmeldetag: 21.10.2009
(51) Int. Cl.: A61K 9/16, A61K 9/20, A61P 5/20

(54) **Schmelzgranuliertes Cinacalcet**

(71) Anmelder: Ratiopharm GmbH, 89079 Ulm (DE)
(72) Erfinder: Paetz, Jana, 82272 Moorenweis (DE); Muskulus, Frank, 88471 Laupheim (DE)
(74) Vertreter: TER MEER - STEINMEISTER & PARTNER GbR

(57) **Zusammenfassung**

Die Erfindung betrifft ein Intermediat, erhältlich durch Schmelzextrusion von (i) Cinacalcet oder eines pharmazeutisch verträglichen Salzes davon, mit (ii) einem Matrixformer sowie orale Darreichungsformen, insbesondere Tabletten enthaltend die erfindungsgemäßen Intermediate. Die Erfindung betrifft ferner ein Verfahren zur Herstellung der erfindungsgemäßen Tabletten. Schließlich betrifft die Erfindung die Verwendung eines Matrixformers und eines Dochtmittels zur Herstellung von Cinacalcet-Formulierungen, die vorzugsweise unabhängig von den Mahlzeiten verabreicht werden können.

## Beschreibung

Die Erfindung betrifft ein Intermediat, erhältlich durch gemeinsame Schmelzverarbeitung von (i) kristallinem Cinacalcet oder eines pharmazeutisch verträglichen Salzes davon, mit (ii) einem Matrixformer sowie Tabletten enthaltend die erfindungsgemäßen Intermediate. Die Erfindung betrifft ferner ein Verfahren zur Herstellung der erfindungsgemäßen Tabletten. Schließlich betrifft die Erfindung die Verwendung eines Matrixformers und eines Dochtmittels zur Herstellung von Cinacalcet-Formulierungen, die vorzugsweise unabhängig von den Mahlzeiten verabreicht werden können.

N-[(1R)-1-(1-naphthyl)ethyl]-3- [3-(trifluormethyl)phenyl]propan-1-amin ist unter dem INN-Namen "Cinacalcet" bekannt und weist folgende Strukturformel auf:

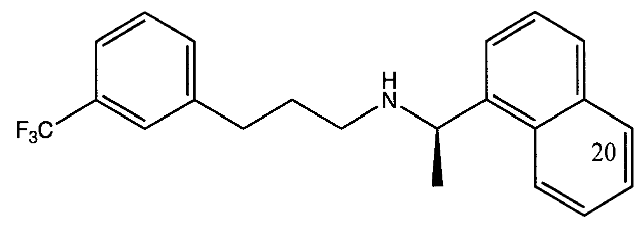

Das Calcimimetikum Cinacalcet dient zur Behandlung des sekundären Hyperparathyreoidismus infolge einer chronischen Niereninsuffizienz. Zudem ist die Substanz zur Behandlung der Hypercalcämie bei Patienten mit Nebenschilddrüsenkarzinom zugelassen.

Synthese und Wirkung von Cinacalcet sind in EP 1 203 761 B1 beschrieben. Patienten mit einer chronischen Nierenerkrankung bekommen infolge ihrer Grunderkrankung häufig eine Nebenschilddrüsen-Überfunktion (sekundärer Hyperparathyreoidismus). Insuffiziente Nieren scheiden mit dem Urin weniger Phosphat aus und bilden weniger aktives Vitamin D3, das für die Aufrechterhaltung eines physiologischen Calciumionen-Blutspiegels notwendig ist. Wenn der Calciumionenspiegel sinkt, wird in den Nebenschilddrüsen vermehrt Parathormon ausgeschüttet. Die Parathormon-Überproduktion bewirkt wiederum, dass Calciumionen aus den Knochen mobilisiert wird und die Knochen brüchiger werden. Cinacalcet bindet an die calciumempfindlichen Rezeptoren an der Oberfläche der Nebenschilddrüsenzellen. Dadurch wird die Empfindlichkeit des Rezeptors gegenüber extrazellulären Calciumionen gesteigert und ein höherer Calciumspiegel im Blut als tatsächlich vorhanden simuliert. Als Folge sinkt die Parathormon-Sekretion, damit wird weniger Calcium aus dem Knochen freigesetzt.

Cinacalcet ist durch Sprühtrocknung auch in amorpher Form erhältlich, siehe WO 2008/000422 A1. Wirkstoffe in amorpher Form zeigen jedoch häufig nachteilige Eigenschaften im Hinblick auf die Lagerstabilität.

Die WO 2008/064202 beschreibt Cinacalcet-haltige Zusammensetzungen mit verzögerter Freisetzung. Darreichungsformen mit verzögerter Freisetzung werden üblicherweise für spezielle Anwendungen verwendet. Für eine Vielzahl von Anwendungen sind jedoch Darreichungsformen mit sofortiger Freisetzung wünschenswert.

Die derzeitig vermarkteten Filmtabletten sind Tabletten mit sofortiger Freisetzung (= Immediate-Release Tabletten) und in WO 2005/034928 beschrieben. Die Tabletten enthalten Cinacalcet in mikronisierter Form mit einem Wirkstoffanteil von ungefähr 18 %. Dabei sollten die Filmtabletten mit oder kurz nach einer Mahlzeit eingenommen werden, da sich die Bioverfügbarkeit bei gleichzeitiger Nahrungsaufnahme um 50 bis 80 Prozent erhöht und erst dann akzeptabel ist.

Mit der Mikronisierung von Cinacalcet gehen jedoch einige Nachteile einher. Zunächst resultiert die Mikronisierung in einem Wirkstoff mit unerwünscht niedriger Fließfähigkeit. Ferner ist der mikronisierte Wirkstoff schwieriger zu verpressen und gelegentlich kommt es zu ungleichmäßiger Wirkstoffverteilung innerhalb der zu verpressenden pharmazeutischen Formulierung. Durch die starke Vergrößerung der Oberfläche während der Mikronisierung nimmt zudem die Oxidationsempfindlichkeit des Wirkstoffs zu.

Aufgabe der vorliegenden Erfindung war es daher, die vorstehend genannten Nachteile zu überwinden. Es soll der Wirkstoff in einer Form bereitgestellt werden, die eine gute Fließfähigkeit aufweist und eine gute Verpressung ermöglicht. Ferner soll eine gleichmäßige Verteilung des Wirkstoffs gewährleistet sein. Eine Mikronisierung des Wirkstoffs soll vermieden werden.

Weiterhin soll der Wirkstoff in einer Form bereitgestellt werden, die gute Löslichkeit bei zeitgleich guter Lagerstabilität gewährleistet. Auch nach 2-jähriger Lagerung (bzw. 3-monatiger Lagerung unter Stressbedingungen) soll eine entsprechend gute Löslichkeit erzielbar sein. Es soll eine Verabreichung unabhängig von den Mahlzeiten ermöglicht werden. Unter dem Begriff "Verabreichung unabhängig von den Mahlzeiten" ist zu verstehen, dass der Patient das Arzneimittel zu den Mahlzeiten einnehmen kann, aber nicht zwingend zu den Mahlzeiten einnehmen muss. Insbesondere soll eine Löslichkeit von größer 3 mg/ml, insbesondere von 10 mg/ml erreicht werden. Ferner soll eine Lagerstabilität von 12 Monaten bei 40 °C und 75 % Luftfeuchte erzielt werden. Die Verunreinigungen sollen nach derartiger Lagerung < 2 Gew.-%, insbesondere < 1 Gew.-% sein. Ferner soll es möglich sein, Cinacalcet-Tabletten sowohl mit rascher Zerfallszeit als auch mit vorteilhafter Härte bereit zu stellen.

Alle vorstehend genannten vorteilhaften Eigenschaften sollen zudem bei hohem Wirkstoffanteil (z.B. bei Wirkstoffgehalten von 20 %, 30 %, 40 % und/oder 50 %) erzielbar sein. Insbesondere sollen die vorstehend genannten Eigenschaften zudem bei hohem Wirkstoffanteil und zugleich hoher "Content Uniformity" erzielbar sein.

Die Aufgaben der vorliegenden Erfindung konnten durch ein Intermediat gelöst werden, das durch Schmelzverarbeitung, bevorzugt Schmelzgranulation oder Schmelzextrusion, von Cinacalcet und Matrixformer erhältlich ist, sowie durch Verwendung des Intermediats zur Herstellung von Tabletten mit sofortiger Freisetzung.

Gegenstand der Erfindung ist somit ein Intermediat, erhältlich durch Schmelzverarbeitung von
(i) Cinacalcet oder eines pharmazeutisch verträglichen Salzes davon, mit
(ii) einem Matrixformer.

Grundsätzlich umfasst im Rahmen dieser Anmeldung der Begriff "Cinacalcet" (i) sowohl die vorstehend dargestellte "freie Base" als auch pharmazeutisch verträgliche Salze davon. Hierbei kann es sich um ein oder mehrere Salze handeln, die auch im Gemisch vorliegen können. Unter "Salz" wird hierbei verstanden, dass die Amingruppe des Cinacalcets protoniert wurde, wobei sich ein positiv geladenes Stickstoffatom bildet, das mit einem entsprechenden Gegenanion assoziiert ist.

Bevorzugt werden als Salze Säureadditionssalze verwendet. Beispiele für geeignete Salze sind Hydrochloride, Carbonate, Hydrogencarbonate, Acetate, Lactate, Butyrate, Propionate, Sulfate, Methansulfonate, Citrate, Tartrate, Nitrate, Sulfonate, Oxalate und / oder Succinate.

Besonders bevorzugt handelt es sich im Falle von Cinacalcet bei dem pharmazeutisch verträglichen Salz um Cinacalcet-Hydrochlorid. Ebenfalls besonders bevorzugt handelt es sich bei dem pharmazeutisch verträglichen Salz um Cinacalcet-Carbonat. Weiterhin besonders bevorzugt handelt es sich bei dem pharmazeutisch verträglichen Salz um Cinacalcet-Methansulfonat.

Bei dem verwendeten Cinacalcet (i), bevorzugt bei dem verwendeten Cinacalcet-Hydrochlorid, handelt es sich üblicherweise um kristallines Material. Bevorzugt wurde es nicht mikronisiert. Bevorzugt wird Cinacalcet-Hydrochlorid in polymorpher Form I verwendet. Die polymorphe Form I ist z.B. in WO 2007/62147 offenbart.

Der Begriff "nicht mikronisiertes Cinacalcet" bezieht sich im Rahmen dieser Erfindung auf partikuläres Cinacalcet, das im allgemeinen einen mittleren Teilchendurchmesser (D50) von 20 bis 280 µm, bevorzugt 60 bis 250 µm, mehr bevorzugt von 65 bis 200 µm, noch mehr bevorzugt von 70 bis 125 µm, und insbesondere von 75 bis 110 µm aufweist.

Der Ausdruck "mittlerer Teilchendurchmesser" bezieht sich im Rahmen dieser Erfindung auf den D50-Wert des volumenmittleren Teilchendurchmessers, der mittels Laserdiffraktometrie bestimmt wurde. Insbesondere wurde zur Bestimmung ein Mastersizer 2000 von Malvern Instruments verwendet. Alle Messbedingungen werden wie auf Seiten 9 und 10 von WO 2005/034928 beschrieben gewählt, d.h. Nassmessung, 1750 rpm, Span^{®} 85 als Dispergiermittel, Auswertung gemäß der Fraunhofer Methode. Der mittlere Teilchendurchmesser, der auch als D50-Wert der integralen Volumenverteilung bezeichnet wird, wird im Rahmen dieser Erfindung als der Teilchendurchmesser definiert, bei dem 50 Volumen-% der Teilchen einen kleineren Durchmesser haben als der Durchmesser, der dem D50-Wert entspricht. Ebenso haben dann 50 Volumen-% der Teilchen einen größeren Durchmesser als der D50-Wert.

Analog wird als D10-Wert der Teilchendurchmesser definiert, bei dem 10 Volumen-% der Teilchen einen kleineren Durchmesser haben als der Durchmesser, der dem D10-Wert entspricht. Ebenso wird als D90-Wert der Teilchendurchmesser definiert, bei dem 90 Volumen-% der Teilchen einen kleineren Durchmesser haben als der Durchmesser, der dem D90-Wert entspricht.

Das nicht mikronisierte Cinacalcet weist ferner üblicherweise D10-Werte von 1 bis 50 µm, mehr bevorzugt von 1 bis 30 µm, und insbesondere von 2 bis 25 µm auf. Weiterhin weist das nicht mikronisierte Cinacalcet üblicherweise D90-Werte von 200 bis 800 µm, mehr bevorzugt von 250 bis 700 µm, und insbesondere von 300 bis 600 µm auf.

Kristallines Cinacalcet liegt üblicherweise in Form von Nadeln vor. Die Charakterisierung mittels volumenmittlerem Teilchendurchmesser ist daher gegebenenfalls nicht spezifisch genug.

Es hat sich gezeigt, dass eine exaktere Charakterisierung von vorteilhaft verwendbarem Cinacalcet, insbesondere Cinacalcet-Hydrochlorid, durch die Beschreibung der spezifischen Oberfläche erfolgen kann.

In einer bevorzugten Ausführungsform wird (i) kristallines Cinacalcet oder ein pharmazeutisch verträgliches Salz davon mit einer spezifischen Oberfläche von 0,01 bis 12 m²/g, mehr bevorzugt von 0,1 bis 8 m²/g, insbesondere von 0,1 bis 7 m²/g, eingesetzt.

Die spezifische Oberfläche wird im Rahmen dieser Erfindung gemäß Gasadsorptionsmethode, insbesondere mittels BET-Methode, bestimmt.

In einer bevorzugten Ausführungsform weist das verwendete Cinacalcet (i), insbesondere das Cinacalcet-Hydrochlorid, einen Wassergehalt von 0,01 bis 0,20 Gew.-%, mehr bevorzugt von 0,02 bis 0,10 Gew.-%, auf. Der Restwassergehalt wird nach der Karl Fischer Methode bestimmt, wobei ein Coulometer bei 160 °C verwendet wird. Bevorzugt wird ein Metrohm 831 KF Coulometer mit einer Titrierzelle ohne Diaphragma verwendet. Üblicherweise wird eine Probe von 20 mg Cinacalcet analysiert.

Es hat sich gezeigt, dass ein höherer Wassergehalt die Fließfähigkeit negativ beeinflussen würde und damit bei einem hohen Wirkstoffgehalt (*drug load*) auch die Gleichförmigkeit des Gehalts (*Content Uniformity*).

Bei dem "Matrixformer" (ii) handelt es sich im Rahmen dieser Erfindung im Allgemeinen um einen Stoff, welcher beim Erwärmen über den Schmelzpunkt, insbesondere bei einem Schmelzgranulations- oder Schmelzextrusionsprozess, verformbar und in der Lage ist, partikuläres Cinacalcet einzubetten, d.h. eine Matrix für partikuläres Cinacalcet zu bilden. Der Matrixformer weist somit bevorzugt ein thermoplastisches Verhalten auf, d.h. es handelt sich um einen thermoplastischen Matrixformer. Der Matrixformer ist zudem ein Stoff, der in Lage ist, sich beim Extrusionsvorgang an Cinacalcet oder Salzen davon (chemisch oder physikalisch) anzulagern und die Hydrophilität der Oberfläche zu erhöhen.

Bei dem Matrixformer (ii) kann es sich um hydrophile Polymere, insbesondere um hydrophile thermoplastische Polymere handeln. Darunter sind Polymere zu verstehen, die hydrophile Gruppen aufweisen. Beispiele für geeignete hydrophile Gruppen sind Hydroxy, Amino, Carboxy, Sulfonat. Ferner weist das zur Herstellung des Intermediats verwendbare hydrophile Polymer bevorzugt ein gewichtsmittleres Molekulargewicht von 1.000 bis 150.000 g/mol auf, mehr bevorzugt von 2.000 bis 90.000 g/mol, insbesondere 3.000 bis 75.000 g/mol, auf. Im Rahmen dieser Anmeldung wird das gewichtsmittlere Molekulargewicht bevorzugt mittels Gel-Permeationschromatographie bestimmt.

Es ist bevorzugt, dass die als Matrixformer verwendeten Polymere im Wesentlichen keine Emulgatorwirkung zeigen. Das heißt, der verwendete Matrixformer sollte bevorzugt keine Kombination aus hydrophilen und hydrophoben Gruppen (insbesondere hydrophoben Fettsäuregruppen) aufweisen. Ferner ist es bevorzugt, dass das erfindungsgemäße Intermediat keine Polymere enthält, die ein gewichtsmittleres Molekulargewicht von mehr als 150.000 g/mol aufweisen. Derartige Polymere beeinflussen in der Regel die Auflöseeigenschaften in unerwünschter Weise.

Wird das als Matrixformer verwendete Polymer in Wasser in einer Menge von 2 Gew.-% gelöst, so zeigt die resultierende Lösung bevorzugt eine Viskosität von 0,1 bis 8 mPa/s, mehr bevorzugt von 0,5 bis 7 mPa/s, insbesondere von 1 bis 6 mPa/s, gemessen bei 25 °C und gemäß Ph. Eur., 6. Auflage, Kapitel 2.2.10 bestimmt.

Darüber hinaus besitzt das als Matrixformer verwendete hydrophile Polymer eine Glasübergangstemperatur (Tg) oder einen Schmelzpunkt von 25 °C bis 200 °C, mehr bevorzugt von 40 °C bis 170 °C. Die Glasübergangstemperatur ist die Temperatur, bei der das hydrophile Polymer beim Abkühlen spröde und beim Erhitzen weich wird. Dies bedeutet, dass hydrophile Polymere bei Temperaturen über der Glasübergangstemperatur (T_{g}) weich werden und ohne zu brechen plastisch verformbar werden. Die Glasübergangstemperatur oder der Schmelzpunkt werden mittels eines Mettler-Toledo^{®} DSC1 bestimmt, wobei eine Aufheizrate von 10 °C pro Minute und eine Kühlrate von 15 °C pro Minute angewandt werden. Die Bestimmungsmethode basiert im Wesentlichen auf Ph.Eur. 6.1, Kapitel 2.2.34. Für die Bestimmung der T_{g} wird das Polymer zweimal erhitzt (d.h. erhitzt, abgekühlt, erhitzt).

Ferner umfasst der Matrixformer (ii) auch feste, nicht-polymere Verbindungen, die bevorzugt polare Seitengruppen aufweisen.

Das erfindungsgemäße Intermediat kann beispielsweise folgende hydrophile Polymere als Matrixformer umfassen: Polysaccharide, wie Hydroxypropylmethylcellulose (HPMC), Polyvinylpyrrolidon, Polyvinylalkohol, Polymere der Acrylsäure und deren Salze, Polyacrylamid, Polymethacrylate, Vinylpyrrolidon-Vinylacetat-Copolymere (beispielsweise Kollidon^{®} VA64, BASF), Polyalkylenglykole, wie Polypropylenglykol oder bevorzugt Polyethylenglykol, Co-blockpolymere des Polyethylenglykols, insbesondere Co-blockpolymere aus Polyethylenglykol und Polypropylenglykol (Pluronic^{®}, BASF) sowie Gemische aus den genannten Polymeren.

Bevorzugt verwendet wird als Matrixformer (ii) Hydroxypropylmethylcellulose (HPMC), bevorzugt mit einem gewichtsmittleren Molekulargewicht von 20.000 bis 90.000 g/mol und/oder bevorzugt einem Anteil an Methylgruppen von 10 bis 35 %; Hydroxypropylcellulose (HPC), bevorzugt mit einem gewichtsmittleren Molekulargewicht von 40.000 bis 100.000 g/mol, Polyvinylpyrrolidon, bevorzugt mit einem gewichtsmittleren Molekulargewicht von 10.000 bis 60.000 g/mol, insbesondere 12.000 bis 40.000 g/mol, Copolymer aus Vinylpyrrolidon und Vinylacetat, insbesondere mit einem gewichtsmittleren Molekulargewicht von 40.000 bis 75.000 g/mol, Polyethylenglykol, insbesondere mit einem gewichtsmittleren Molekulargewicht von 2.000 bis 50.000 g/mol, Polyoxyethylenalkylether und/oder Polyvinylalkohol, bevorzugt mit einem gewichtsmittleren Molekulargewicht von 1.000 bis 50.000 g/mol, werden verwendet.

Als Matrixformer (ii) besonders bevorzugt verwendet werden Co-Blockpolymere aus Polyethylenglykol und Polypropylenglykol, d.h. Polyoxyethylen Polyoxypropylen-Blockpolymerisate. Bevorzugt weisen diese ein gewichtsmittleres Molekulargewicht von 1.000 bis 20.000 g/mol, mehr bevorzugt von 1.500 bis 12.500 g/mol, insbesondere 5.000 bis 10.000 g/mol, auf. Diese Blockpolymerisate sind bevorzugt erhältlich durch Kondensation von Propylenoxid mit Propylenglykol und nachfolgender Kondensation des entstandenen Polymers mit Ethylenoxid. Das heißt, bevorzugt liegt der Ethylenoxidanteil als "Endblock" vor. Bevorzugt weisen die Blockpolymerisate ein Gewichtsverhältnis von Propylenoxid zu Ethylenoxid von 50 : 50 bis 95 : 5, mehr bevorzugt von 70 : 30 bis 90 : 10, auf. Die Blockpolymerisate weisen bevorzugt eine Viskosität bei 25 °C von 200 bis 2000 mPas, mehr bevorzugt von 500 bis 1500 mPas, insbesondere von 800 bis 1200 mPas, auf.

Im Rahmen dieser Erfindung können auch Gemische der vorstehend genannten Matrixformer eingesetzt werden. In einer möglichen Ausführungsform wird beispielsweise ein Gemisch aus Polyvinylpyrrolidon und Polyoxyethylen Polyoxypropylen-Blockpolymerisaten eingesetzt.

In einer bevorzugten Ausführungsform enthält das erfindungsgemäße Intermediat Cinacalcet oder ein pharmazeutisch verträgliches Salz davon, bevorzugt in nicht mikronisierter Form, und Matrixformer, wobei das Gewichtsverhältnis von Wirkstoff (i) zu Matrixformer (ii) 5 : 1 bis 1 : 5, mehr bevorzugt 3 : 1 bis 1 : 3, noch mehr bevorzugt 2 : 1 bis 1 : 2, insbesondere ungefähr 1 : 1 beträgt.

Es ist bevorzugt, dass Art und Menge des Matrixformers so gewählt werden, dass mindestens 50 % der Oberfläche der resultierenden Intermediatteilchen mit Matrixformer bedeckt sind, mehr bevorzugt mindestens 60 % der Oberfläche, besonders bevorzugt mindestens 80 % der Oberfläche, insbesondere mindestens 95 % der Oberfläche.

Im Rahmen dieser Erfindung ist es besonders bevorzugt, dass Cinacalcet (i) und Matrixformer (ii) gemeinsam "schmelzverarbeitet" werden. Bevorzugt wird hierbei die Schmelzverarbeitung als Schmelzextrusion oder mehr bevorzugt als Schmelzgranulation durchgeführt. Der Schmelzverarbeitung können, wie nachstehend beschrieben, ferner noch weitere pharmazeutische Hilfsstoffe, wie z.B. Sprengmittel und Dochtmittel, beigesetzt werden. Sofern Sprengmittel und Dochtmittel (quasi intragranulär) im erfindungsgemäßen Intermediat enthalten sind, werden diese im Rahmen dieser Anmeldung als Komponenten (iii-int) beziehungsweise (iv-int) bezeichnet. Sofern Sprengmittel und Dochtmittel (quasi extragranulär) in der erfindungsgemäßen oralen Darreichungsform (als Komponente (β)) enthalten sind, werden diese im Rahmen dieser Anmeldung als Komponenten (iii-ex), beziehungsweise (iv-ex), bezeichnet. Sofern auf die Gesamtmenge an Sprengmittel oder Dochtmittel (d.h. sowohl extra- als auch intragranulär) Bezug genommen werden soll, wird die Bezeichnung (iii) beziehungsweise (iv) verwendet.

Die Schmelzverarbeitung kann, wie nachstehend beschrieben, in üblichen Schmelzverarbeitungsvorrichtungen erfolgen.

Die Schmelzbedingungen werden bei Einsatz von kristallinem Cinacalcet üblicherweise so gewählt, dass Cinacalcet in kristallinem Zustand verbleibt.

Das erfindungsgemäße Intermediat findet bei der Herstellung einer oralen Darreichungsform Verwendung. Bei der oralen Darreichungsform handelt es sich z.B. um Kapseln, Pulver oder Granulat zur Abfüllung in Sachets oder Tabletten. Die Herstellung von Tabletten ist hierbei bevorzugt. Besonders bevorzugt findet das erfindungsgemäße Intermediat zur Herstellung einer Tablette mit sofortiger Freisetzung (im Englischen als "Immediate-Release Tablet" bekannt) Verwendung.

Gegenstand der Erfindung ist daher eine orale Darreichungsform, insbesondere eine Tablette mit sofortiger Freisetzung, enthaltend
(α) erfindungsgemäßes Intermediat und
(β) pharmazeutische Hilfsstoffe.

Hierbei handelt es sich um die dem Fachmann bekannten Hilfsstoffe (β), insbesondere solche, die im Europäischen Arzneibuch beschrieben sind.

Beispiele für verwendete Hilfsstoffe (β) sind Sprengmittel, Trennmittel, Füllstoffe, Zusätze zur Verbesserung der Pulverfließfähigkeit, Gleitmittel, Netzmittel, und/oder Schmiermittel.

Das Verhältnis von Wirkstoff zu Hilfsstoffe wird bevorzugt so gewählt, dass die resultierenden Formulierungen
5 bis 60 Gew.-%, mehr bevorzugt 20 bis 45 Gew.-% Cinacalcet, und
40 bis 95 Gew.-%, mehr bevorzugt 55 bis 80 Gew.-%, pharmazeutisch verträgliche Hilfsstoffe enthalten. Bevorzugt handelt es sich, wie vorstehend erläutert, hierbei um nicht-mikronisiertes, kristallines Cinacalcet.

Bei diesen Angaben wird die Menge an Matrixformer, die zur Herstellung des erfindungsgemäßen Intermediats verwendet wurde, als Hilfsstoff gerechnet. Das heißt, die Menge an Wirkstoff bezieht sich auf die Menge an Cinacalcet, die in der fertigen Formulierung enthalten ist.

In einer bevorzugten Ausführungsform enthält die erfindungsgemäße Tablette 1 bis 40 Gew.-%, 5 bis 35 Gew.-%, mehr bevorzugt 10 bis 30 Gew.-%, besonders bevorzugt 15 bis 25 Gew.-% Sprengmittel (iii), bezogen auf das Gesamtgewicht der Formulierung. Als Sprengmittel werden im Allgemeinen Stoffe bezeichnet, die den Zerfall einer Darreichungsform, insbesondere einer Tablette, nach Einbringen in Wasser, beschleunigen. Geeignete Sprengmittel sind z.B. organische Sprengmittel wie Carrageenan, Cellulosen und Cellulosederivate: Croscarmellose, Stärken und Stärkederivate: Natriumcarboxymethylstärke, Polysaccharide: Soja-Polysaccharide, Alginate und Crospovidon. Zudem können anorganische Sprengmittel wie Bentonite verwendet werden. Ebenso können alkalische Sprengmittel verwendet werden. Unter alkalischen Sprengmitteln sind Sprengmittel zu verstehen, die beim Lösen in Wasser einen pH-Wert von mehr als 7,0 erzeugen. Es können auch Gemische der vorstehend genannten Sprengmittel verwendet werden.

Besonders bevorzugt wird Crospovidon und/oder Croscarmellose als Sprengmittel, insbesondere in den oben genannten Mengen, verwendet.

In einer bevorzugten Ausführungsform enthält die erfindungsgemäße Tablette 0 bis 35 Gew.-%, 1 bis 30 Gew.-%, mehr bevorzugt 5 bis 25 Gew.-%, besonders bevorzugt 10 bis 20 Gew.-% Dochtmittel (iv), bezogen auf das Gesamtgewicht der Formulierung.

Im Allgemeinen ist ein Dochtmittel (iv) ein Mittel mit der Fähigkeit, eine biologische Flüssigkeit (bevorzugt Wasser) in einen Feststoff zu ziehen (bevorzugt in die Intermediate (i), bevorzugt mittels Physisorption). Physisorption wird definiert als Form der Adsorption, bei welcher die Flüssigkeitsmoleküle an die Oberfläche des Dochtmittels anhaften können, bevorzugt mittels van-der-Waals-Bindung zwischen der Oberfläche des Dochtmittels und dem adsorbierten flüssigen Molekül (bevorzugt Wasser). Normalerweise bewirkt ein Dochtmittel dies mit oder ohne Aufquellen. Normalerweise wird ein nicht quellendes Dochtmittel, das Wasser anzieht, letztendlich ein Volumen haben, das im Wesentlichen aus dem Volumen des Dochtmittels und der Wassermenge besteht, das es anzieht. Im Allgemeinen wird ein quellendes Dochtmittel ein Volumen aufweisen, das im Wesentlichen aus dem Volumen des Dochtmittels, der Wassermenge, die es anzieht, und einem zusätzlichen Volumen, verursacht durch sterische und molekulare Kräfte, besteht.

Bevorzugt verursacht das Dochtmittel (iv) in dem erfindungsgemäßen Intermediat oder in der erfindungsgemäßen Tablette die Bildung von Kanälen oder Poren. Dies erleichtert das Eindringen der Wassermoleküle in die Intermediate, insbesondere durch Physisorption. Daher besteht die Funktion des Dochtmittels darin, Wasser an die Oberflächen innerhalb der Intermediate zu transportieren, um dadurch Kanäle in oder ein Netzwerk auf einer vergrößerten Oberfläche zu schaffen.

Beispiele von verwendeten Dochtmitteln sind: mikrokristalline Cellulose, silifizierte mikrokristalline Cellulose, kolloidales Siliciumdioxid, Kaolin, Titandioxid, pyrogene Kieselsäure, Aluminium, Niacinamid, m-Pyrol, Bentonit, Magnesium-Aluminium-Silicat, Polyester, Polyethylen, oder Mischungen davon. Bevorzugt enthalten die Dochtmittel der pharmazeutischen Zusammensetzung der vorliegenden Erfindung Cellulose und Cellulosederivate, wie z.B. silifizierte mikrokristalline Cellulose, kolloidales Siliciumdioxid, und Mischungen davon. Die bevorzugt verwendete silifizierte mikrokristalline Cellulose ist kommerziell erhältlich unter dem Handelsnamen Prosolv^{®} und weist einen Siliciumdioxidgehalt von 1 bis 3 Gew.-%, bevorzugt von 2 Gew.-% auf.

Die erfindungsgemäße orale Darreichungsform, insbesondere Tablette, kann ferner Füllstoffe (v) enthalten. Unter Füllstoffen sind im Allgemeinen Stoffe zu verstehen, die zur Bildung des Tablettenkörpers bei Tabletten mit geringen Wirkstoffmengen (z.B. kleiner 60 Gew.-%) dienen. Das heißt, Füllstoffe erzeugen durch "Strecken" der Wirkstoffe eine ausreichende Tablettiermasse. Füllstoffe dienen üblicherweise also dazu, eine geeignete Tablettengröße zu erhalten.

Beispiele für bevorzugte Füllstoffe sind Lactose, Lactosederivate, Stärke, Stärkederivate, behandelte Stärke, Chitin, Cellulose und Derivate davon, Calciumphosphat, Saccharose, Calciumcarbonat, Magnesiumcarbonat, Magnesiumoxid, Maltodextrin, Calciumsulfat, Dextrate, Dextrin und/oder Dextrose, hydrogeniertes Pflanzenöl..

Ebenfalls können als Füllstoffe Zuckeralkohole und/oder Disaccharide wie Mannitol, Sorbitol, Xylitol, Isomalt, Glucose, Fructose, Maltose und Gemische daraus verwendet werden. Der Begriff Zuckeralkohole umfasst hier auch Monosaccharide.

Füllstoffe werden üblicherweise in einer Menge von 1 bis 80 Gew.-%, mehr bevorzugt von 5 bis 50 Gew.-%, insbesondere von 20 bis 40 Gew.-%, bezogen auf das Gesamtgewicht der Formulierung verwendet.

Die erfindungsgemäße Tablette kann ferner Zusätze zur Verbesserung der Pulverfließfähigkeit enthalten. Ein Beispiel für einen Zusatz zur Verbesserung der Pulverfließfähigkeit ist disperses Siliciumdioxid, z.B. bekannt unter dem Handelsnamen Aerosil^{®}. Bevorzugt wird Siliciumdioxid mit einer spezifischen Oberfläche von 50 bis 400 m²/g, bestimmt nach Gasadsorption gemäß Ph. Eur., 6. Auflage 2.9.26., verwendet.

Zusätze zur Verbesserung der Pulverfließfähigkeit werden üblicherweise in einer Menge von 0,1 bis 5 Gew.-%, z.B. 1,5 bis 4 Gew.-%, bezogen auf das Gesamtgewicht der Formulierung verwendet.

Ferner können Schmiermittel verwendet werden. Schmiermittel dienen im Allgemeinen zur Verringerung der Gleitreibung. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat (Pruv^{®}) und/oder Magnesiumstearat dar.

Schmiermittel werden üblicherweise in einer Menge von 0,1 bis 5 Gew.-%, bevorzugt 1,0 bis 3 Gew.-%, bezogen auf das Gesamtgewicht der Formulierung, verwendet.

Weiterhin können Trennmittel verwendet werden. Unter Trennmitteln werden üblicherweise Stoffe verstanden, welche die Agglomeration im Kernbett vermindern. Beispiele sind Talkum, Silicagel, Polyethylenglykol (bevorzugt mit 2.000 bis 10.000 g/mol gewichtsmittlerem Molekulargewicht) und/oder Glycerolmonostearat.

Es liegt in der Natur von pharmazeutischen Hilfsstoffen, dass diese teilweise mehrere Funktionen in einer pharmazeutischen Formulierung wahrnehmen. Im Rahmen dieser Erfindung gilt zur unzweideutigen Abgrenzung daher bevorzugt die Fiktion, dass ein Stoff, der als ein bestimmter Hilfsstoff verwendet wird, nicht zeitgleich auch als weiterer pharmazeutischer Hilfsstoff eingesetzt wird. Beispielsweise wird Sorbitol - sofern als Füllstoff eingesetzt - nicht auch zusätzlich als Matrixformer eingesetzt. Ebenfalls wird beispielsweise mikrokristalline Cellulose - sofern als Dochtmittel eingesetzt - nicht auch zusätzlich als Füllstoff eingesetzt (obwohl mikrokristalline Cellulose auch eine Füllwirkung erzeugt).

In einer bevorzugten Ausführungsform enthält die erfindungsgemäße Tablette (bezogen auf das Gesamtgewicht des Tablettenkerns) folgende Bestandteile:
15 bis 40 Gew.-% Cinacalcet (i)
15 bis 35 Gew.-% Matrixformer (ii)
5 bis 40 Gew.-%, bevorzugt 15 bis 40 Gew.-% Füllstoff (v)
15 bis 35 Gew.-% Sprengmittel (iii),
0 bis 30 Gew.-% Dochtmittel (iv) und
1 bis 4 Gew.-% Schmiermittel.

In einer alternativen bevorzugten Ausführungsform enthält die erfindungsgemäße Tablette (bezogen auf das Gesamtgewicht des Tablettenkerns) folgende Bestandteile:
mehr als 40 bis 60 Gew.-% Cinacalcet (i)
15 bis 35 Gew.-% Matrixformer (ii)
0 bis 10 Gew.-% Füllstoff (v)
15 bis 35 Gew.-% Sprengmittel (iii),
0 bis 20 Gew.-% Dochtmittel (iv) und
1 bis 4 Gew.-% Schmiermittel.

Die erfindungsgemäßen Tabletten enthalten bevorzugt keine Polymere, welche zu einer verzögerten Freisetzung führen. Insbesondere ist es bevorzugt, dass die erfindungsgemäßen Tabletten keine Polymere enthalten, die ein Molekulargewicht von mehr als 150.000 g/mol aufweisen.

Gegenstand der Erfindung ist ferner ein Verfahren zur Herstellung der erfindungsgemäßen Tabletten, umfassend die Schritte
(a) Bereitstellung und bevorzugt Vermischen von (i) kristallinem Cinacalcet oder dessen pharmazeutisch verträglichen Salzen mit (ii) einem Matrixformer, sowie gegebenenfalls weiteren pharmazeutischen Hilfsstoffen;
(b) Schmelzverarbeitung, bevorzugt Schmelzextrusion oder insbesondere Schmelzgranulation, zu einem Intermediat;
(c) gegebenenfalls Granulierung des Intermediats;
(d) Kompression der resultierenden Intermediate (bevorzugt der aus Schritt (c) resultierenden Granulate) zu Tabletten, gegebenenfalls unter Zusatz weiterer pharmazeutischer Hilfsstoffe; und
(e) gegebenenfalls Befilmung der Tabletten.

Grundsätzlich finden alle Erläuterungen, die vorstehend zu bevorzugten Ausführungsformen des erfindungsgemäßen Intermediats ausgeführt wurden, auch auf das erfindungsgemäße Verfahren Anwendung.

In einer bevorzugten Ausführungsform werden in Schritt (a) des erfindungsgemäßen Verfahrens (i) Cinacalcet, bevorzugt kristallines Cinacalcet oder dessen pharmazeutisch verträglichen Salze mit (ii) einem Matrixformer sowie gegebenenfalls weiteren - vorstehend beschriebenen - pharmazeutischen Hilfsstoffen (β) bereitgestellt und bevorzugt vermischt.

Wie vorstehend erwähnt, umfasst der Matrixformer bevorzugt kein Polymer mit einem gewichtsmittleren Molekulargewicht von mehr als 150.000 g/mol. Gleiches gilt für die pharmazeutischen Hilfsstoffe, die in Schritt (a) (und/oder auch in Schritt (d)) des erfindungsgemäßen Verfahrens zugegeben werden.

Die Vermischung kann in üblichen Mischern erfolgen. Beispielsweise ist ein Turbula^{®} T 10B (Bachofen AG, Schweiz) geeignet. Die Mischzeit beträgt üblicherweise 1 Minute bis 1 Stunde, bevorzugt 5 Minuten bis 20 Minuten.

In einer bevorzugten Ausführungsform werden in Schritt (a)
100 % des eingesetzten Cinacalcet,
100 % des eingesetzten Matrixformers,
gegebenenfalls 20 bis 70 % des eingesetzten Füllstoffs,
gegebenenfalls 20 bis 70 % des eingesetzten Dochtmittels, und
gegebenenfalls 30 bis 70 % des eingesetzten Sprengmittels, und
gegebenenfalls 10 bis 40 % des eingesetzten Schmiermittels
vermischt. Die verbleibenden optionalen Mengen an Füllstoff, Sprengmittel und Schmiermittel werden anschließend in Schritt (d) zugegeben.

In Schritt (b) des erfindungsgemäßen Verfahrens wird das Gemisch aus Schritt (a) zum erfindungsgemäßen Intermediat schmelzverarbeitet, d.h. bevorzugt schmelzextrudiert oder schmelzgranuliert. Im Rahmen der Schmelzverarbeitung (b) wird Cinacalcet (i) mit dem - bevorzugt thermoplastischen - Matrixformer (ii) so verarbeitet, dass Cinacalcet im Matrixmaterial eingebettet ist. In diesem Zusammenhang ist es bevorzugt, dass die Schmelzbedingungen so gewählt werden, dass der Matrixformer geschmolzen oder angeschmolzen wird, der Wirkstoff aber in festem Zustand verbleibt. Bevorzugt wird Cinacalcet in kristalliner Form (insbesondere Cinacalcet-Hydrochlorid in kristalliner Form I) verwendet und die Schmelzbedingungen bevorzugt so gewählt, dass der Wirkstoff in kristalliner Form, insbesondere kristalliner Form I, erhalten bleibt.

Bevorzugt liegt die bei der Schmelzverarbeitung gewählte Temperatur 10 °C unter bis 10 °C über des Schmelzpunkts des Matrixformers, bevorzugt mit der Maßgabe, dass die gewählte Temperatur mindestens 10 °C unterhalb der Schmelztemperatur des verwendeten Cinacalcets liegt.

Die Schmelzverarbeitung kann bevorzugt als Schmelzgranulation oder als Schmelzextrusion durchgeführt werden.

In einer bevorzugten Ausführungsform erfolgt eine Schmelzgranulation. Das Schmelzverfahren läuft in diesem Fall bevorzugt über einen Intensivmischer mit beheizbarer Manteleinheit, beispielsweise kann ein Diosna^{®} P1-6 vorteilhaft verwendet werden. Hierbei wird üblicherweise die Mischung aus den Bestandteilen (i) und (ii) ohne Aufheizen des Mantels trocken vorgemischt (= Schritt a) und erst im zweiten Schritt (b) durch Zuschalten des beheizbaren Mantels, bevorzugt unter Rühren, erwärmt. Die Erwärmung wird bevorzugt fortgeführt bis eine Zunahme der Kraftaufnahme zu beobachten ist. Anschließend wird granuliert und abgekühlt.

In einer bevorzugten Ausführungsform erfolgt eine Schmelzextrusion. Hierbei handelt es sich um ein kontinuierliches Verfahren (chargenunabhängig), wobei die Vormischung und Granulierung nicht zeitlich sequentiell erfolgt, sondern in einem Produktionsschritt. Ein bevorzugtes Verfahren zur Herstellung des Schmelzextrudats stellt die Schmelzextrusion über einen Zweischneckenextruder (z.B. Leistritz^{®} micro 18) dar. Vorteil hierbei ist die Einstellung eines Temperaturgradienten, abhängig vom gewählten Matrixformer, der die Verweilzeit des Cinacalcet unter hohen Temperaturen deutlich verringert. Der Temperaturgradient liegt üblicherweise zwischen 80 - 190 °C und wird bevorzugt so gewählt, dass das Cinacalcet nach der Verarbeitung noch kristallin vorliegt.

Im optionalen Schritt (c) des erfindungsgemäßen Verfahrens wird das Extrudat granuliert. Die Granulierung kann vor, während oder nach dem Abkühlen erfolgen. Bevorzugt erfolgt die Granulierung bereits im Rahmen der Schmelzverarbeitung. So können beispielsweise die Schritte (b) und (c) auch als ein Verfahrensschritt aufgefasst werden.

In einer bevorzugten Ausführungsform werden die Granulierungsbedingungen (im Schritt (b) oder im Schritt (c)) so gewählt, dass die resultierenden Teilchen (Granulate) eine gewichtsmittlere Teilchengröße (D50-Wert) von 75 bis 600 µm aufweisen, mehr bevorzugt von 120 bis 500 µm, noch mehr bevorzugt von 150 bis 400 µm, insbesondere von 200 bis 350 µm. Die gewichtsmittlere Teilchengröße wird mittels Siebanalyse bestimmt (unter Verwendung einer Retsch^{®} AS 2000, Amplitude 1,5 sec., Intervall 10 min., Probeneinwaage 15,8 g).

Weiterhin werden die Granulierbedingungen bevorzugt so gewählt, dass die resultierenden Granulate eine Schüttdichte von 0,3 bis 0,85 g/ml, mehr bevorzugt 0,4 bis 0,8 g/ml, insbesondere 0,5 bis 0,7 g/ml aufweisen. Der Hausner-Faktor liegt üblicherweise im Bereich von 1,02 bis 1,3, mehr bevorzugt von 1,03 bis 1,25 und insbesondere von 1,04 bis 1,15. Unter "Hausner-Faktor" wird hierbei das Verhältnis von Stampfdichte zu Schüttdichte verstanden. Die Bestimmung von Schütt- und Stampfdichte erfolgt gemäß USP 24, Test 616 "Bulk Density and Tapped Density".

In Schritt (d) des erfindungsgemäßen Verfahrens werden die in Schritt (c) erhaltenen Granulate zu Tabletten verpresst, d.h. es erfolgt eine Kompression zu Tabletten. Die Kompression kann mit im Stand der Technik bekannten Tablettiermaschinen wie Exzenterpressen oder Rundlaufpressen erfolgen. Im Falle von Rundlaufpressen wird üblicherweise eine Presskraft von 2 bis 40 kN, bevorzugt von 2,5 bis 35 kN, angewandt. Beispielsweise wird die Presse Fette^{®} 102i (Fette GmbH, Deutschland) verwendet. Im Falle von Exzenterpressen wird üblicherweise eine Presskraft von 1 bis 20 kN, bevorzugt von 2,5 bis 10 kN, angewandt. Beispielsweise wird die Korsch^{®} EKO verwendet.

Verfahrensschritt (d) erfolgt bevorzugt in Abwesenheit von Lösungsmitteln, insbesondere organischen Lösungsmitteln, d.h. als Trockenkompression.

In Schritt (d) des erfindungsgemäßen Verfahrens können den Intermediaten aus Schritt (b) oder (c) pharmazeutische Hilfsstoffe (β) zugegeben werden. Hierbei wird auf die vorstehend ausgeführten Erläuterungen zu geeigneten Hilfsstoffen verwiesen.

Gegenstand der Erfindung ist nicht nur das erfindungsgemäße Verfahren, sondern auch die mit diesem Verfahren hergestellten Tabletten.

Bei den durch das erfindungsgemäße Verfahren hergestellten Tabletten kann es sich um Tabletten, die unzerkaut geschluckt werden (unbefilmt oder bevorzugt befilmt), handeln. Ebenfalls kann es sich um Kautabletten oder um Disperstabletten handeln. Unter "Disperstablette" wird hierbei eine Tablette zur Herstellung einer wässrigen Suspension zum Einnehmen verstanden.

Im Falle von Tabletten, die unzerkaut geschluckt werden, ist es bevorzugt, dass diese mit einer Filmschicht im Schritt (e) des erfindungsgemäßen Verfahrens überzogen werden. Die vorstehend genannten Verhältnisse von Wirkstoff zu Hilfsstoff beziehen sich jedoch auf die unlackierte Tablette.

Für die Befilmung werden bevorzugt makromolekulare Stoffe verwendet, beispielsweise modifizierte Cellulosen, Polymethacrylate, Polyvinylpyrrolidon, Polyvinylacetatphthalat, Zein und/oder Schellack.

Bevorzugt verwendet wird HPMC, insbesondere HPMC mit einem gewichtsmittleren Molekulargewicht von 10.000 bis 150.000 g/mol und/oder einem durchschnittlichen Substitutionsgrad an -OCH₃-Gruppen von 1,2 bis 2,0.

Die Schichtdicke des Überzugs beträgt bevorzugt 1 bis 100 µm mehr bevorzugt 2 bis 80 µm.

Die Tablettierbedingungen werden bevorzugt so gewählt, dass die resultierenden Tabletten ein Verhältnis von Tablettenhöhe zu Gewicht von 0,005 bis 0,3 mm/mg, besonders bevorzugt 0,05 bis 0,2 mm/mg aufweisen.

Ferner weisen die resultierenden Tabletten bevorzugt eine Härte von 70 bis 200 N, besonders bevorzugt von 100 bis 150 N auf, insbesondere wenn das Tablettengewicht mehr als 200 mg beträgt. Sofern das Tablettengewicht 200 mg oder weniger beträgt, weisen die resultierenden Tabletten bevorzugt eine Härte von 30 bis 100 N, besonders bevorzugt von 40 bis 70 N, auf. Die Härte wird gemäß Ph.Eur. 6.0, Abschnitt 2.9.8 bestimmt.

Zudem zeigen die resultierenden Tabletten bevorzugt eine Friabilität von kleiner 3 %, besonders bevorzugt von kleiner 1 %, insbesondere von kleiner 0,8 %, auf. Die Friabilität wird gemäß Ph.Eur. 6.0, Abschnitt 2.9.7 bestimmt.

Schließlich weisen die erfindungsgemäßen Tabletten üblicherweise eine "Content Uniformity" von 95 bis 105 % vom durchschnittlichen Gehalt, bevorzugt von 98 bis 102 %, insbesondere von 99 bis 101 % vom durchschnittlichen Gehalt, auf. Die "Content Uniformity" wird gemäß Ph. Eur.6.0, Abschnitt 2.9.6. bestimmt.

Das Freisetzungsprofil der erfindungsgemäßen Tabletten weist gemäß USP-Methode (Paddle, 900 ml 0,1 N HCl, pH 1,2, 37 °C, 75 UpM) nach 10 Minuten üblicherweise einen freigesetzten Gehalt von mindestens 30 %, bevorzugt mindestens 50 %, insbesondere mindestens 70 % auf. Sofern diese Parameter erfüllt sind, werden die Tabletten als Tabletten mit sofortiger Freisetzung betrachtet.

Die vorstehenden Angaben zu Härte, Friabilität, Content Uniformity und Freisetzungsprofil beziehen sich hierbei bevorzugt auf die unbefilmte Tablette.

Alternativ zur Verpressung in Tabletten können die in Schritt (c) des erfindungsgemäßen Verfahrens resultierenden Granulate auch - gegebenenfalls unter Zusetzung weiterer pharmazeutischer Hilfsstoffe - zu einer partikulären Darreichungsform verarbeitet werden, beispielsweise durch Abfüllung in Kapseln oder in Sachets.

Gegenstand der Erfindung ist somit eine orale Darreichungsform enthaltend Cinacalcet, Matrixformer und Sprengmittel zur Behandlung des Hyperparathyreoidismus, wobei die Verabreichung unabhängig von den Mahlzeiten erfolgt. In einer bevorzugten Ausführungsform wird hierfür Sprengmittel in einer Menge von 10 bis 30 Gew.-% verwendet, bezogen auf das Gesamtgewicht der oralen Darreichungsform. In einer weiteren bevorzugten Ausführungsform wird hierfür ein Polyoxyethylen Polyoxypropylen-Blockpolymerisat als Matrixformer verwendet, insbesondere wie vorstehend näher beschrieben. In einer weiteren bevorzugen Ausführungsform beträgt der Gehalt an Cinacalcet 20 bis 60 Gew.-%, insbesondere 40 bis 60 Gew.-%.

Die Erfindung soll anhand der nachfolgenden Beispiele veranschaulicht werden.

### BEISPIELE

### Beispiel 1

### Kern:

Cinacalcet-Hydrochlorid (D50 101 µm): 33,0 mg
Polyoxyethylen Polyoxypropylen-Blockpolymerisat (Mw ca. 8350): 30,0 mg
Sorbitol (Füllstoff): 47,0 mg
Natriumstearylfumarat: 7,00 mg
Crospovidon: 28,0 mg

### Film:

Opadry^{®} AMB 6,40 mg

Das Herstellverfahren umfasste folgende Schritte:
■ Cinacalcet HCl und Polyoxyethylen Polyoxypropylen-Blockpolymerisat, 20 mg wurden unter vorsichtigem Erwärmen bis zum Schmelzpunkt des Polymers granuliert
■ die resultierenden Intermediate wurde gesiebt (Maschenweite 0,6 mm) und weitere 10 min gemischt,
■ das Granulat wurde zusammen mit Crospovidon und Sorbitol 10 min lang gemischt,
■ Natriumstearylfumarat wurde durch Sieben hinzugefügt (Maschenweite 0,3 mm) und weitere 5 min lang gemischt,
■ die erhaltene Mischung wurde zu Tabletten verpresst (9,7 x 5 r 3,6; 4,5 kN; 42 N), und
■ die Tabletten wurden mit einer Opadry^{®} AMB-Lösung überzogen.

Die resultierenden Tabletten zeigten vorteilhafte Löslichkeitseigenschaften, die nach Lagerung über drei Monate (bei 40 °C, 75 % Luftfeuchte) erhalten blieben, siehe Beispiel 2.

### Vergleichsbeispiel 1

Zum Vergleich wurde Tabletten gemäß WO 2005/34928 A1 (Absatz [0057]), enthaltend 30 mg mikronisiertes Cinacalcet-HCl mittels Feuchtgranulation hergestellt. Das Löslichkeitsverhalten wurde in Beispiel 2 untersucht.

### Beispiel 2

Das in-vitro Löslichkeitsverhalten von (unbefilmten) Tabletten gemäß Beispiel 1 und Vergleichsbeispiel 1 wurde gemäß USP (Paddle, 900 ml 0,1 N HCl, pH 1,2, 37 °C, 75 rpm) vor und nach Lagerung (40 °C, 75 % rel. Luftfeuchte) untersucht.

| **Beispiel** | **Rührzeit** | **Menge ohne Lagerung** | **Menge nach 2 Wochen** | **Menge nach 4 Wochen** | **Menge nach 12 Wochen** |
|---|---|---|---|---|---|
| Beispiel 1 | 15 | 60,2 | 90,4 | 92,2 | 100,8 |
| Vergleichsbeispiel 1 | 15 | 83,1 | 86,5 | 84,3 | 83,7 |
| | | | | | |

Die Messung zeigt, dass die erfindungsgemäßen Tabletten ein sehr gutes Löslichkeitsverhalten, insbesondere nach Lagerung, aufweisen, wobei eine Mikronisierung des Wirkstoffs vermieden werden konnte.

### Beispiel 3

Es wurde eine Gehaltsbestimmung sowie eine Messung der Verunreinigung mittels HPLC Methode von Tabletten gemäß Beispiel 1 vor und nach Lagerung (40 °C. 75 % rel. Luftfeuchte) durchgeführt.
HPLC-Parameter:
Säule: X-Bridge C18 150 x 4,6 mm, 3,5 µm
Durchfluss: 0,9 ml/min.
Säulentemperatur: 60°C
Injektionsvolumen: 2 µl
Elutionsmittel A: 25 mmol/l KH₂PO₄ *H₂O pH 3.00 ± 0,05
Elutionsmittel B: Acetonitril

| Pumpengradient: | Zeit [min] | % B |
|---|---|---|
| | 0 | 25 |
| | 3 | 25 |
| | 22 | 65 |
| | 25 | 25 |

Wellenlänge: 225 nm
Proben-Lösungsmittel: Wasser / Acetonitril 50/50
Probenkonzentrierung: 450 µg/ml

| | **ohne Lagerung** | **Nach 4 Wochen** | **Nach 12 Wochen** |
|---|---|---|---|
| Gehalt | 96,3 | 95,52 | 96,09 |
| Verunreinigung total | 0,03 | 0,07 | 0,09 |

Die Bestimmung zeigt, dass die erfindungsgemäßen Tabletten eine sehr gute Lagerstabilität aufweisen.

## Patentansprüche

1. Intermediat, erhältlich durch Schmelzverarbeitung von
(i) Cinacalcet oder eines pharmazeutisch verträglichen Salzes davon, mit
(ii) einem Matrixformer.

2. Intermediat nach Anspruch 1, wobei kristallines Cinacalcet oder ein pharmazeutisch verträgliches Salz davon, eingesetzt wird.

3. Intermediat nach Anspruch 2, wobei die Schmelzverarbeitungsbedingungen so gewählt werden, dass Cinacalcet in kristallinem Zustand verbleibt.

4. Intermediat nach einem der Ansprüche 1 bis 3, wobei als Matrixformer hydrophile Polymere mit einem gewichtsmittleren Molekulargewicht von 1.000 g/mol bis 150.000 g/mol eingesetzt werden.

5. Intermediat nach einem der Ansprüche 1 bis 4, wobei als Matrixformer Polyoxyethylen Polyoxypropylen-Blockpolymerisate, bevorzugt mit einem gewichtsmittleren Molekulargewicht von 1.500 bis 12.500 g/mol verwendet werden.

6. Intermediat nach Anspruch 5, wobei das Gewichtsverhältnis von Komponente (i) zu Komponente (ii) 1 : 5 bis 5 : 1 beträgt.

7. Intermediat nach einem der Ansprüche 1 bis 6, enthaltend ferner
(iii-int) Sprengmittel und/oder
(iv-int) Dochtmittel.

8. Orale Darreichungsform, bevorzugt in Form einer Tablette, bevorzugt mit sofortiger Freisetzung, enthaltend
(α) ein Intermediat gemäß einem der Ansprüche 1 bis 7 und
(β) pharmazeutische Hilfsstoffe.

9. Orale Darreichungsform nach Anspruch 8, **dadurch gekennzeichnet, dass** die Komponente (β) Sprengmittel (iii-ex) und/oder Dochtmittel (iv-ex) enthält.

10. Orale Darreichungsform nach Anspruch 9, wobei die Gesamtmenge an Sprengmittel (iii-int) + (iii-ex) 10 bis 30 Gew.-%, bezogen auf das Gesamtgewicht der Formulierung, beträgt.

11. Orale Darreichungsform nach einem der Ansprüche 8 bis 10, wobei die Tablette einen Gehalt an Cinacalcet von 40 bis 60 Gew.-% aufweist.

12. Verfahren zur Herstellung einer oralen Darreichungsform gemäß einem der Ansprüche 8 bis 11 in Form einer Tablette, umfassend die Schritte
(a) Bereitstellen von (i) Cinacalcet oder dessen pharmazeutisch verträglichen Salzen, mit (ii) einem Matrixformer sowie gegebenenfalls weiteren pharmazeutischen Hilfsstoffen;
(b) Schmelzverarbeitung zu einem Intermediat;
(c) gegebenenfalls Granulierung des Intermediats;
(d) Kompression der resultierenden Intermediate zu Tabletten, gegebenenfalls unter Zusatz weiterer pharmazeutischer Hilfsstoffe; und
(e) gegebenenfalls Befilmung der Tabletten.

13. Verfahren gemäß Anspruch 12, wobei die Schmelzbedingungen in Schritt (b) so gewählt werden, dass Cinacalcet in kristallinem Zustand verbleibt.

14. Verfahren gemäß Anspruch 12 oder 13, wobei in Schritt (b) oder (c) Granulate mit einer gewichtsmittleren Teilchengröße von 120 bis 500 µm hergestellt werden.

15. Orale Darreichungsform enthaltend Cinacalcet, Matrixformer, Dochtmittel und Sprengmittel zur Behandlung des Hyperparathyreoidismus, wobei die Verabreichung unabhängig von den Mahlzeiten erfolgt.
